# EUROPEAN PATENT APPLICATION

(11) **EP 1 215 496 A1**
(43) Date of publication of application: **19.06.2002**
(21) Application number: 00957085.4
(22) Date of filing: 11.09.2000
(51) Int. Cl.: G01N 33/543

(54) **KIT FOR DETECTING OR ASSAYING SUBJECT SUBSTANCE AND DETECTION OR ASSAY METHOD**

(30) Priority: 13.09.1999 JP 25877199
(71) Applicant: NISSUI PHARMACEUTICAL CO., LTD., Toshima-ku, Tokyo 170-0002 (JP)
(72) Inventor: OKU, Yuichi Nissui Pharmaceutical Co., Ltd., Yuuki-shi, Ibaraki 307-0036 (JP); KAMIYA, Hisanori Nissui Pharmaceutical Co., Ltd.,, Yuuki-shi Ibaraki 307-0036 (JP); SHINOHARA, Kumiko Nissui Pharmaceutical Co., Ltd., Yuuki-shi, Ibaraki 307-0036 (JP); SIBAHARA, Yusuke Nissui Pharmaceutical Co., Ltd., Yuuki-shi Ibaraki 307-0036 (JP); UESAKA, Yoshihiko Nissui Pharmaceutical Co., Ltd., Yuuki-shi Ibaraki 307-0036 (JP)
(74) Representative: Fiener, Josef
(86) International application number: JP0006187
(87) International publication number: WO0120328

(57) **Abstract**

A kit and a production method therefor are provided, which kit can be produced in a high yield, can easily label ligands and can control the sensitivity of reagents. A kit for detecting or measuring an analyte having bivalent or higher binding capability includes the following receptor I, receptor II, and solid phase conjugate. Receptor I: L1-B1-R1-M including a compound R1-M bound to a compound L1-B1, in which the compound R1-M includes a substance R1 being capable of binding to a substance B1 bound to a marker M, the compound L1-B1 includes a ligand L1 and the substance B1. Receptor II: L2-B2-R2-B3 including a compound L2-B2 previously bound to a compound R2-B3, in which the compound L2-B2 includes a ligand L2 capable of binding to the analyte A and a substance B2 being introduced into the ligand L2 and having different binding capability from the analyte A, the compound R2-B3 includes a substance R2 capable of binding to the substance B2 and being bound to a bond element B3 having different binding. Solid phase conjugate: R3-solid phase including an anti-bond element R3 bound to a solid phase, in which the anti-bond element R3 is capable of binding to the bond element B3.

## Description

### 1. Technical Field

The present invention relates to kits and methods for detecting or measuring an analyte having bivalent or higher binding capability and contained in a liquid sample. The kits each comprise a ligand-marker complex, a ligand-bond element complex, and a solid phase. The ligand-marker complex comprises a ligand which specifically binds to the analyte and is labeled with a marker directly or indirectly. The ligand-bond element complex comprises a ligand and a bond element, which ligand specifically binds to the analyte and which bond element serves to bind the ligand to the solid phase. The solid phase comprises an anti-bond element which allows the solid phase to capture the ligand-bond element complex.

### 2. Background Art

Known methods for detecting or measuring an antibody or another substance in a liquid sample, which substance has bivalent or higher binding capability to a ligand, include the following method. In the method, a ligand labeled with a marker such as an enzyme, and a solid phase bound to a ligand are previously prepared, and the ligand labeled with the marker is allowed to react with an antibody in a liquid sample. The resulting solution mixture is then allowed to react with the ligand bound to the solid phase to thereby determine the presence or absence of the marker bound to the solid phase or to measure the amount thereof (e.g., Japanese Patent Application Publication No. 9-229938). According to this method, the antibody reactive with respect to the identical ligand previously reacts with the labeled ligand and becomes unreactive with the solid phased ligand, and the resulting sensitivity is insufficient in many cases.

For this reason, for example, Japanese Patent No. 2532788 discloses a method for determining the presence or the amount of an immunoreactive analyte (G). In this method, a capture moiety (R) is bound to a solid phase, a first batch (M-I1) of a first particle is bound to a second batch (I2-B) of a second particle through the medium of the immunoreactive analyte (G) and thereby yields a complex (M-I1-G-I2-B). The first batch (M-I1) comprises a first immunological substance (II) bound to a detectable isotope (M) and is dispersed. The second batch (I2-B) comprises a second binding product of a second immunoreactive substance (I2) and a species (B) capable of being captured and is dispersed. The formed complex (M-I1-G-I2-B) is allowed to react with the capture moiety (R) bound to the solid phase to thereby determine the presence or amount of the immunoreactive analyte. According to this method, when the first immunological substance (I1) is identical to the second immunological substance, the first immunological substance (I1) and the detectable isotope (M) are not bound to the immunoreactive analyte (G), but the first immunological substance (I1) and the second immunological substance (I2) concurrently react with the immunoreactive analyte (G). Thus, the method exhibits a high sensitivity.

Japanese Patent Application Publication No. 10-253632 discloses a method for evaluating an analyte based on the presence or absence or the amount of a marker (M). In this method, an oligonucleotide is solid-phased, a ligand (L) labeled with an oligonucleotide (ON') being complementary to the oligonucleotide (ON) (the resulting oligonucleotide-labeled ligand is ON'-L, e.g., ON'-antigen or ON'-antibody) and a marker-labeled ligand (M-L) are allowed to react with the analyte (A) being capable of binding to the ligand (L) and thereby yield a complex (ON'-L-A-L-M). The complex is then bound to the solid phase by complementary binding between oligonucleotides contained in the oligonucleotide-binding solid phase and the complex, respectively, to determine the presence or absence or the amount of the marker (M). This method exhibits a high sensitivity as in the method described in Japanese Patent No. 2532788, since the analyte (A) and the marker-labeled ligand (M-L) do not previously form a complex, but ON'-L and M-L concurrently react with the analyte (A). According to the method in question, plural analytes can concurrently be measured by changing the sequences of the oligonucleotide to be solid-phased and of the oligonucleotide to be bound to the corresponding ligand (L).

To detect or measure an analyte with a high sensitivity as in the methods described in Japanese Patent No. 2532788 and Japanese Patent Application Publication No. 10-253632, a first or second immunoreactive substance or a ligand must be labeled with a bond element of some kind.

However, when the immunoreactive substance or the ligand (L) is directly labeled with the bond element, the ligand (L) changes in physical properties and tends to become insoluble in a purification step of the labeled ligand (L) during labeling process. Accordingly, the recovery of the resulting bond element-labeled ligand (e.g., an antigen labeled with the bond element) significantly decreases in some cases.

When a low molecular weight peptide as a ligand (L) is directly labeled, the labeled peptide can be recovered but may show decreased immunochemical reactivity. This is probably caused by steric hindrance. In addition, when the low molecular weight peptide is to be labeled with a metal colloid or a colored latex, it may not be labeled because of its low molecular weight.

Japanese Patent No. 2501960 discloses a reagent. The reagent comprises a substance P2 (e.g., avidin or streptavidin) that is labeled with a marker and has specific binding capability, a substance P2 that is not labeled with a marker and has specific binding capability, a compound P1-R1, and a solid phase bound to the substance P2 that is not labeled with a marker. The compound P1-R1 comprises a component P1 (e.g., biotin) having monovalent binding capability to P2, and a ligand R1 bound to P1. The reagent cannot significantly be controlled in sensitivity, since the amount of the ligand per molecule of the complex cannot be controlled, or the reaction with the solid phase yields a strong bond derived from the bond between biotin and avidin. The reagent may also react with a trace quantity of an immunity substance originated from another factor and may yield unsuitable conclusions.

Accordingly, an object of the present invention is to provide a kit and a method for detecting or measuring an antibody or another analyte contained in a liquid sample, which analyte has bivalent or higher binding capability to a ligand. The kit can easily detect or measure the analyte, can be prepared in a high yield and can easily label a target substance regardless of whether it is a low molecular weight ligand, a high molecular weight ligand or a ligand constituting part of a protein. The kit can produce a ligand-marker complex in a high yield even when, for example, a ligand of a low molecular weight compound is labeled with a marker. In addition, the amount of the ligand in the kit can be controlled, or the binding capability between the solid phase and the complex can be changed, and therefore the sensitivity of a constitutional reagent can be controlled.

### DISCLOSURE OF INVENTION

The present invention provides the following kits to solve the above problems.

A kit of a first type according to the present invention is a kit for detecting or measuring an analyte having bivalent or higher binding capability. The kit comprises the following receptor I, receptor II, and solid phase conjugate for capturing a complex comprising the receptor I, the receptor II and the analyte:
1) a receptor I represented by a compound L1-B1-R1-M comprising a compound R1-M bound to a compound L1-B1, the compound R1-M comprising a substance R1 bound to a marker M, the substance R1 being capable of binding to a substance B1, the compound L1-B1 comprising a ligand L1 bound to the substance B1, the ligand L1 being capable of binding to the analyte A;
2) a receptor II represented by a compound L2-B2-R2-B3 comprising a compound L2-B2 previously bound to a compound R2-B3, the compound L2-B2 comprising a ligand L2 and a substance B2 introduced into the ligand L2, the ligand L2 being capable of binding to the analyte A, the substance B2 having different binding capability from analyte A, the compound R2-B3 comprising a substance R2 bound to a bond element B3, the substance R2 being capable of binding to the substance B2, the bond element B3 having binding capability different from the substance B2; and
3) solid phase conjugate represented by a compound R3-solid phase, the compound R3-solid phase comprising an anti-bond element R3 bound to a solid phase, the anti-bond element R3 being capable of binding to the bond element B3.

A kit of a second type according to the present invention is a kit for detecting or measuring an analyte A having bivalent or higher binding capability to a ligand L3 constituting part of a protein P. The kit comprises the following receptor I, receptor II, and solid phase conjugate for capturing a complex comprising the receptor I, the receptor II and the analyte:
1) a receptor I represented by a compound P-M comprising the protein P bound to a marker M;
2) a receptor II represented by a compound P-B2-R2-B3 or L3-B2-R2-B3 comprising a compound P-B2 or L3-B2 bound to a compound R2-B3, the compound P-B2 or L3-B2 comprising the protein P or the ligand L3 and a substance B2 introduced into the protein P or the ligand L3, the substance B2 having different binding capability from analyte A, the compound R2-B3 comprising a substance R2 bound to a bond element B3, the substance R2 being capable of binding to the substance B2, the bond element B3 having binding capability different from the substance B2; and
3) solid phase conjugate represented by a compound R3-solid phase, the compound R3-solid phase comprising ananti-bond element R3 bound to a solid phase, the anti-bond element R3 being capable of binding to the bond element B3.

In the kits of the present invention, the solid phase conjugate is at least independent of the receptor II. Specifically, as assembly in the kits of the present invention before measurement, the solid phase conjugate is independent of the receptor II and the receptor I, or the solid phase conjugate is contained in the same system with the receptor I but is independent of the receptor II.

A kit of a third type according to the present invention is a kit for detecting or measuring an analyte A having bivalent or higher binding capability, the kit comprising the following receptor I, receptor II, and solid phase conjugate for capturing a complex comprising the receptor I, receptor II and analyte:
1) a receptor I represented by a compound L1-B1-R1-M comprising a compound R1-M bound to a compound L1-B1, the compound R1-M comprising a substance R1 bound to a marker M, the substance R1 being capable of binding to a substance B1, the compound L1-B1 comprising a ligand L1 bound to the substance B1, the ligand L1 being capable of binding to the analyte A;
2) a receptor II represented by a compound L2-B3, the compound L2-B3 comprising a ligand L2 previously bound to a bond element B3, the ligand L2 being capable of binding to the analyte A, the bond element B3 having different binding capability from analyte A; and
3) solid phase conjugate represented by a compound R3-solid phase, the compound R3-solid phase comprising an anti-bond element R3 bound to a solid phase, the anti-bond element R3 being capable of binding to the bond element B3.

The analyte A having bivalent or higher binding capability in the kits of the present invention can be selected from the group consisting of DNA, RNA, antigen, and antibody.

In the kits of the present invention, the ligand L1 and the ligand L2 may be the same substance (e.g., both are avidin or both are streptavidin) or may be different substances having different sequences (e.g., both are DNAs or RNAs having different sequences or are avidin and streptavidin).

The ligands L (L1, L2, L3) in the kits of the present invention can be selected from the group consisting of DNA, RNA, antigen, antibody, lectin, glycoprotein, and sugar.

The substance R1 and/or the substance R2 in the kits of the present invention can be selected from the group consisting of streptavidin, avidin, antigen, antibody, DNA, RNA, lectin, glycoprotein, and sugar.

The substance R1 and the substance R2 in the kits of the present invention may be the same substance (e.g., both are avidin or both are streptavidin) or different substances (e.g., avidin and streptavidin).

The substance B1 and/or the substance B2 in the kits of the present invention can be a substance selected from the group consisting of DNA, RNA, antigen, antibody, lectin, glycoprotein, and sugar. Biotin is typically used as the substance B1 and/or the substance B2 in an embodiment of the kits of the present invention. In this case, the substance R1 and the substance R2 are both avidin, both streptavidin, or a combination of avidin and streptavidin.

In the kits of the present invention, the binding capability between the substance B1 and the substance R1 or between the substance B2 and the substance R2 is preferably represented by a dissociation constant of from 10⁻⁸ o 10⁻¹⁶ (M).

The marker in the kits of the present invention may be a substance selected from the group consisting of coloring dye, fluorescent dye, luminescent substance, metal colloid, latex, liposome, radioactive isotope, enzyme, DNA, and RNA.

The solid phase for use in the present invention is preferably a substance selected from the group consisting of polystyrene, nitrocellulose, nylon, cellulose, and glass.

A method for detecting or measuring an analyte A according to the present invention using the kit of the first type is a method for detecting or measuring an analyte A having bivalent or higher binding capability. The method comprises the steps of:
bringing
1) the analyte A into contact with:
2) a receptor I represented by a compound L1-B1-R1-M comprising a compound R1-M bound to a compound L1-B1, the compound R1-M comprising a substance R1 bound to a marker M, the substance R1 being capable of binding to a substance B1, the compound L1-B1 comprising a ligand L1 bound to the substance B1, the ligand L1 being capable of binding to the analyte A; and
3) a receptor II represented by a compound L2-B2-R2-B3 comprising a compound L2-B2 previously bound to a compound R2-B3, the compound L2-B2 comprising a ligand L2 and a substance B2 introduced into the ligand L2, the ligand L2 being capable of binding to the analyte A, the substance B2 having different binding capability from analyte A, the compound R2-B3 comprising a substance R2 bound to a bond element B3, the substance R2 being capable of binding to the substance B2, the bond element B3 having binding capability different from the substance B2, to react with one another to thereby form a complex;
4) allowing a solid phase conjugate to capture the formed complex, the solid phase conjugate represented by a compound R3-solid phase, the compound R3-solid phase comprising an anti-bond element R3 bound to a solid phase, the anti-bond element R3 being capable of binding to the bond element B3, and
5) detecting or measuring the marker M in the captured complex.

A method for detecting or measuring an analyte A of the present invention using the kit of the second type is a method for detecting or measuring an analyte A having bivalent or higher binding capability to a ligand L3 constituting part of a protein P. The method includes the steps of:
bringing
1) the analyte A into contact with:
2) a receptor I represented by a compound P-M comprising the protein P bound to a marker M; and
3) a receptor II represented by a compound P-B2-R2-B3 or L3-B2-R2-B3 comprising a compound P-B2 or L3-B2 bound to a compound R2-B3, the compound P-B2 or L3-B2 comprising the protein P or the ligand L3 and a substance B2 introduced into the protein P or the ligand L3, the substance B2 having different binding capability from analyte A, the compound R2-B3 comprising a substance R2 bound to a bond element B3, the substance R2 being capable of binding to the substance B2, the bond element B3 having binding capability different from the substance B2, to react with one another to thereby form a complex,
4) allowing a solid phase conjugate to capture the formed complex, the solid phase conjugate represented by a compound R3-solid phase, the compound R3-solid phase comprising an anti-bond element R3 bound to a solid phase, the anti-bond element R3 being capable of binding to the bond element B3, and
5) detecting or measuring the marker M in the captured complex.

A method for detecting or measuring an analyte A of the present invention using the kit of the third type is a method for detecting or measuring an analyte A having bivalent or higher binding capability. The method includes the steps of:
bringing
1) the analyte A into contact with:
2) a receptor I represented by a compound L1-B1-R1-M comprising a compound R1-M bound to a compound L1-B1, the compound R1-M comprising a substance R1 bound to a marker M, the substance R1 being capable of binding to a substance B1, the compound L1-B1 comprising a ligand L1 bound to the substance B1, the ligand L1 being capable of binding to the analyte A; and
3) a receptor II represented by a compound L2-B3, the compound L2-B3 comprising a ligand L2 previously bound to a bond element B3, the ligand L2 being capable of binding to the analyte A, the bond element B3 having different binding capability from analyte A, to react with one another to thereby form a complex,
4) allowing a solid phase conjugate to capture the formed complex, the solid phase conjugate represented by a compound R3-solid phase, the compound R3-solid phase comprising an anti-bond element R3 bound to a solid phase, the anti-bond element R3 being capable of binding to the bond element B3, and
5) detecting or measuring the marker M in the captured complex.

In the three methods for detecting or measuring the analyte A, the complex comprising the elements 1), 2) and 3) may be formed in one step by concurrently proceeding reactions or may sequentially be formed in plural steps by changing the order of bringing the elements into contact with one another.

Embodiments of the compound L1-B1-R1-M (receptor I) for use in the kit of the present invention of the first type include a compound comprising streptavidin or avidin as the substance R1 which is bound to a metal colloid or enzyme as the marker M to form R1-M, and a biotin-bound ligand as the compound L1-B1 bound to R1-M by action of specific binding reaction between biotin and avidin or streptavidin. This compound is a novel substance.

The kit of the first type of the present invention can be applied to the case where the ligand L1 is soluble in water and can advantageously be applied specifically to the case where the ligand L1 is highly insoluble. When such a highly insoluble ligand L1 is directly labeled, the resulting complex may become insoluble owing to the insolubility of the ligand L1 and may cause a nonspecific reaction. However, by binding the ligand with the substance R1 or R2 having relatively high hydrophilicity, such as avidin or streptavidin, the resulting complex represented by the compound L1-B1-R1-M (receptor I) or the compound L2-B2-R2-B3 (receptor II) can become hydrophilic as a whole and can prevent occurrence of a nonspecific reaction.

In this connection, when the biotin-avidin reaction is used for preparing individual complexes constituting the kit, the condition for the preparation of the complexes can be maintained constant regardless of the type of the ligand L1. This configuration can save extra time and effort. According to the present invention, the complex L2-B2-R2-B3 (receptor II) is prepared in advance. The amount of the ligand per molecule of the complex can therefore be controlled and the sensitivity of the reagent can thereby be controlled.

The kit of the second type of the present invention is suitable when the ligand L2 is not a low molecular weight substance but a water-soluble polymer such as a water-soluble protein. When such a protein is used as the ligand L2, the ligand L2 can directly be labeled with the marker M.

The kit of the third type of the present invention is suitable for measuring an oligonucleotide in DNA or RNA as the analyte A. In this case, the ligand L1 is an oligonucleotide at least having a complementary sequence with respect to a sequence of one end of the oligonucleotide analyte A, and the ligand L2 is an oligonucleotide at least having a complementary sequence with respect to a sequence of the other end of the analyte A. In addition, the sequence of an end of the ligand L2 opposite to that bound to the analyte A is at least complementary with respect to the sequence of the oligonucleotide of the DNA or RNA bound to the solid phase.

### BRIEF DESCRIPTION OF THE DRAWINGS

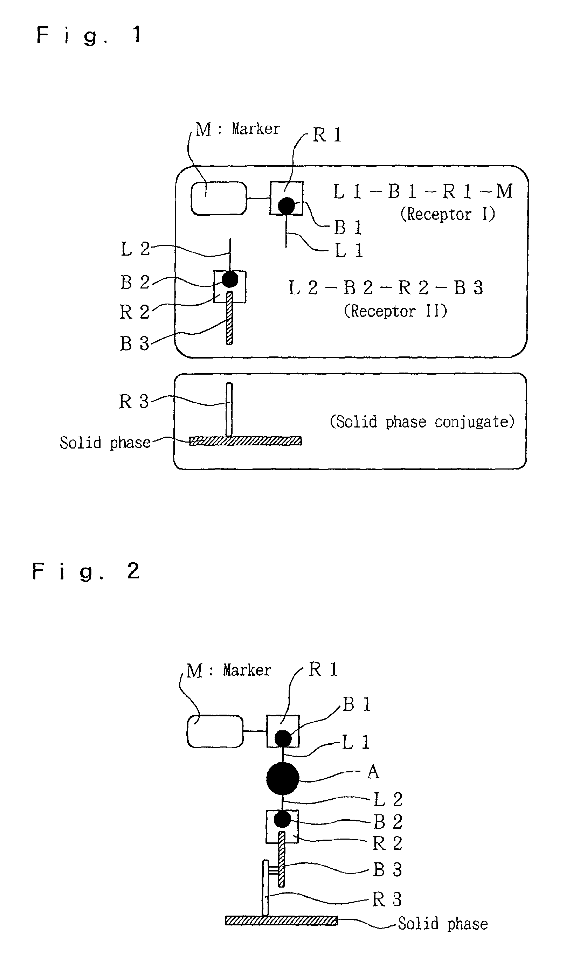

Fig. 1 illustrates an embodiment of components of the kit of the first type of the present invention. During storage of the kit, a component in one frame is stored in configuration separated from with components in the other frame.

In Fig. 2, the analyte A is captured by the solid phase using the kit of the first type of the present invention.

Fig. 3 is a flowchart for the preparation of reagent components of the kit of the first type of the present invention. Specifically, it is a preparation flowchart for the receptor I represented by the compound L1-B1-R1-M and for the receptor II represented by the compound L2-B2-R2-B3.

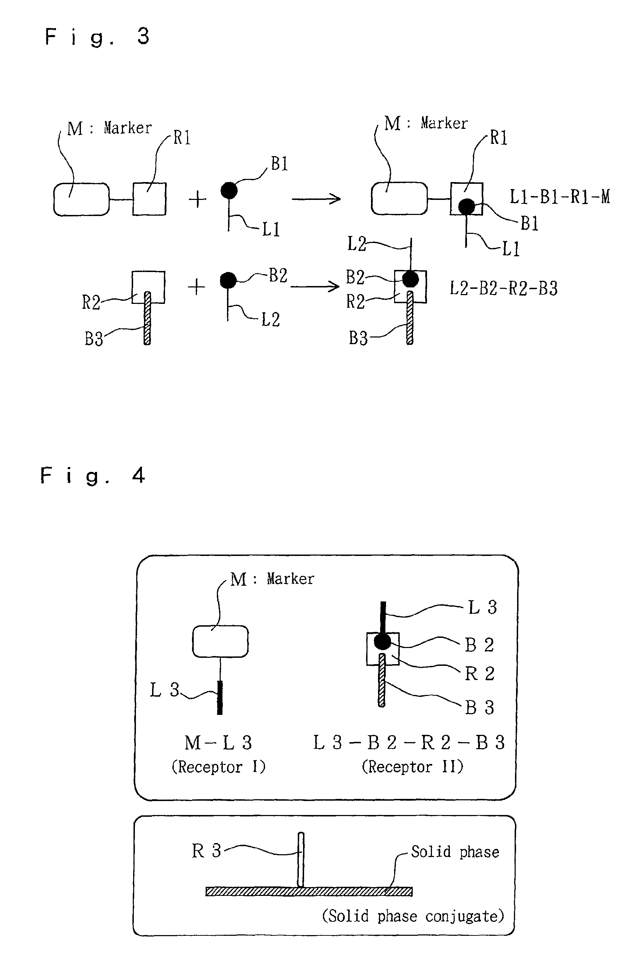

Fig. 4 illustrates an embodiment of components of the kit of the second type of the present invention. During storage of the kit, a component in one frame is stored in configuration separated from with components in the other frame.

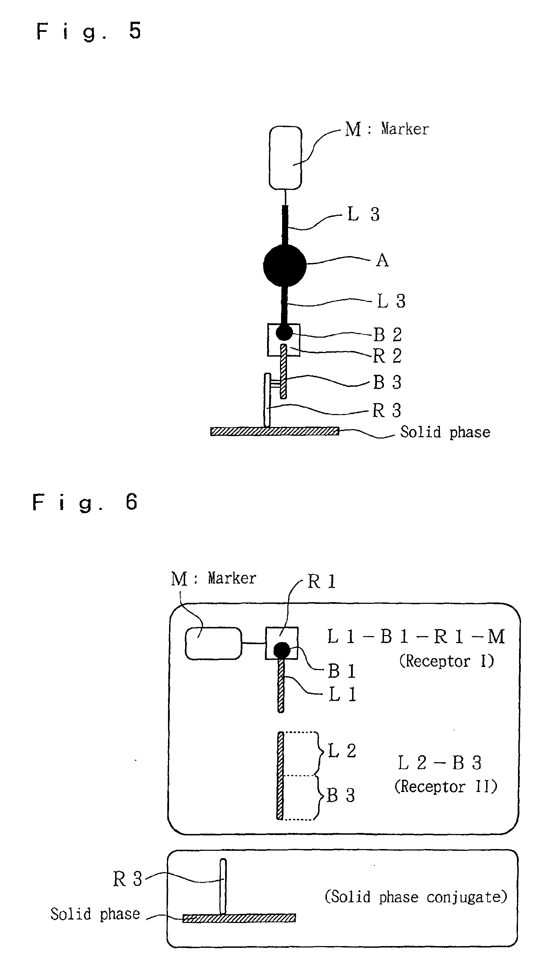

In Fig. 5, the analyte A is captured by the solid phase using the kit of the second type of the present invention.

Fig. 6 illustrates an embodiment of components of the kit of the third type of the present invention. During storage of the kit, a component in one frame is stored in configuration separated from with components in the other frame.

In Fig. 7, the analyte A is captured by the solid phase using the kit of the third type of the present invention.

In Fig. 8, the kit of the present invention comprising the ligand L1 and the ligand L2 having different sequences from each other is allowed to react with the analyte A to thereby allow the solid phase conjugate to capture a complex comprising the receptor I, the receptor II and the analyte.

### BEST MODE FOR CARRYING OUT THE INVENTION

The technical significance of the present invention will be illustrated with reference to several embodiments of the invention.

Avidin or streptavidin satisfactorily binds to biotin with an association constant of 10⁻¹⁵ (M), 100 folds or more higher than those of general immunoreactions. It has been reported that this bond dissociates within 15 minutes under the condition of 6-8 M guanidine, pH 1.5 and 120° C (Green, N. : Purification of Avidin, in Methods in Enzymology, XVIII, (McCormick, D., and Wright, L., eds.), Academic Press, NY, 414 (1970)) to thereby form a complex that is very stable at room temperature.

An example of the kit of the first type of the present invention comprising avidin or streptavidin as R1 or R2 will be illustrated. In this kit, use can be made of a receptor I represented by the compound L1-B1-R1-M or a receptor II represented by the compound L2-B2-R2-B3. The compound L1-B1-R1-M comprises the ligand L1 bound to the marker M and the compound L2-B2-R2-B3 comprises the ligand L2 bound to an oligonucleotide as the bond element B3, each by action of strong bond between avidin or streptavidin as R1 or R2 and biotin or another component as B1 or B2.

In the preparation of the receptor I, initially, biotin as the substance B1 is introduced into the ligand L1 to form the compound L1-B1, and the marker M is bound to avidin or streptavidin as the substance R1 to form the compound R1-M. Avidin or streptavidin R1 and biotin B1 in these compounds are then bound to each other and thereby very easily and stably yield the complex (receptor I) represented by the compound L1-B1-R1-M. The above-prepared complex is substantially a substance comprising the ligand L1 labeled with the marker M.

The compound L1-B1-R1-M (receptor 1) is the complex prepared according to the above method and comprises the marker M bound to, for example, avidin or streptavidin (R1) and the biotinylated ligand (the compound L1-B1) bound to avidin or streptavidin (R1). Accordingly, even when the ligand L1 is a low molecular weight peptide or another substance that is susceptible to steric hindrance, it becomes less subject to such hindrance, as the marker M is bound to the ligand L1 through the medium of the high molecular weight avidin or streptavidin (R1). In addition, even when the ligand L1 is a substance that cannot directly be bound to the marker M, the method can reliably produce the complex of the ligand L1 and the marker M.

When the ligand L3 is a relatively stable substance that is resistant to steric hindrance, that is, when the ligand L3 constitutes part of a protein, the marker M can directly be bound to the ligand L3 or to the protein P containing the ligand L3, and the resulting compound P-M can be used as a ligand-marker complex (receptor I) (the kit of the second type of the present invention).

When the substance B1 is identical to the substance B2, and the ligand L1 is identical to the ligand L2, the kit of the first type of the present invention can easily be prepared according to the following method. To prepare the receptor II, i.e., the compound (L2-B2-R2-B3) comprising the ligand L2 compounded with the bond element B3, the substance R2 is previously bound to the bond element B3 such as an oligonucleotide ON and thereby yields the compound R2-ON. The compound L1-B1 prepared in the preparation of the receptor I (L1-B1-R1-M), wherein L1-B1 is the same compound with L2-B2, is then allowed to react with the compound R2-ON and thereby yields a complex (receptor II) represented by a compound L2-B2-R2-ON. This method can prepare the complex comprising the ligand L2 and the nucleotide ON (bond element B3).

This method can reliably prepare the complex of the ligand L2 and the nucleotide ON (bond element B3) through the medium of the substance R2 (e.g., a substance selected from the group consisting of streptavidin, avidin, antigen, antibody, DNA, RNA, lectin, glycoprotein, and sugar) and the substance B2 even when the ligand L2 is a low molecular weight peptide or another substance that is diffcult to labeling.

To compose the kit of the first type of the present invention, the compound R1-M and the compound R2-B3 (e.g., R2-ON) are prepared in advance, and the compound L1-B1 is used in common in the case where the compound L1-B1 is identical to the compound L2-B2, and the complex of the ligand L1 and the marker M, i.e., the receptor I represented by L1-B1-R1-M, and the complex of the ligand L2 and the bond element B3 (e.g., the oligonucleotide ON), i.e., the receptor II represented by the compound L2-B2-R2-B3, are then prepared as shown in the flowchart in Fig. 3. By this procedure, the kit of the first type of the present invention can very easily be prepared.

Avidin or streptavidin for use as the substance R1 or the substance R2 has four binding sites per molecule with respect to biotin as the substance B1 or the substance B2. Accordingly, the introduction ratio of the ligand L1 or the ligand L2 can be changed in the range from one to four per molecule of avidin or streptavidin, and the reactivity and sensitivity can therefore be controlled by controlling the introduction ratio.

When the ligand L1 or the ligand L2 is a highly hydrophobic substance, and the marker M is directly introduced into the ligand L1 or the bond element B3 such as the oligonucleotide ON is directly introduced into the ligand L2, the physical properties further change, the hydrophobicity is further enhanced and thereby causes nonspecific bonds in many cases. However, when the ligand-biotin is bound to the avidin-marker or the avidin-bond element as in the kit of the first type of the present invention and when the ligand-marker complex or the ligand-bond element complex is prepared, the hydrophobicity of the ligand can be mitigated by action of binding with avidin having high hydrophilicity, thus eliminating nonspecific reactions due to hydrophobicity. In addition, the reactivity can also be controlled in this procedure by changing the introduction ratio of avidin to the ligand-biotin.

The above explanation is mainly on the kit of the first type of the present invention but can also be applied to the kit of the second type of the present invention as in the kit of the first type.

The invented kits and methods for detection or quantification use the bond element B3 bound to the anti-bond element R3 to allow the solid phase to capture the complex. By employing combinations of substances having various binding capabilities, the intensity of the binding capability can be varied, and thereby the sensitivity of the reagent can be controlled. For example, the binding reaction between lectin and a sugar exhibits reactivity much lower than that of the biotin-avidin binding reaction. Accordingly, when the lectin and sugar are used as B3 and R3, the resulting kit does not react unless an antigen or antibody in a much more amount is contained in the sample than that in a kit using the biotin-avidin reaction.

When the bond element B3 and the anti-bond element R3 are oligonucleotides of RNA and/or DNA at least having partly complementary sequences and are bound to each other by action of their complementarity, the intensity of binding capability can be controlled, and thereby the sensitivity of the reagent can be controlled by changing the lengths and/or homology of the complementary sequences of the oligonucleotides.

The invented kits and methods for detection or quantification can simultaneously detect or measure plural analytes by preparing plural combinations of oligonucleotides to be solid-phased and corresponding oligonucleotides to be bound to corresponding ligands and selecting ligands that can specifically be bound to the plural analytes corresponding to the individual combinations to thereby constitute a kit.

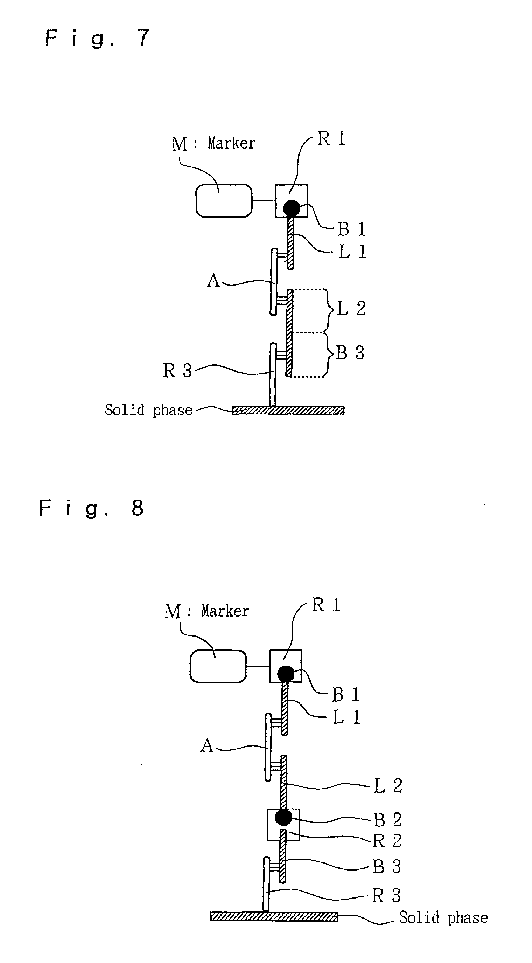

With reference to Fig. 8, the kit of the present invention comprising a ligand L1 and a ligand L2 having different sequences from each other is used and is allowed to react with an analyte A, and thereby the complex comprising the receptor I, the receptor II and the analyte is captured by the solid phase conjugate. Specifically, the analyte A shown in Fig. 8 is an oligonucleotide of an RNA or DNA. One oligonucleotide and the other having complementary sequences to both ends of the aforementioned oligonucleotide are used as the ligands L1 and L2, respectively. The components of the kit constituting the embodiment shown in Fig. 8 are the same with those of the kit of the first type of the present invention.

### EXAMPLE 1

### Synthesis of Oligonucleotides

The following oligonucleotides each having an amino group at five prime end were synthesized by using a DNA synthesizer available from The Perkin-Elmer Corporation, respectively. Some of the oligonucleotides were synthesized by Sawady Technology Co., Ltd.
Amino group-GAA TTC CCG GGG ATC CGT CG (hereinafter referred to as "Pair 1+")
Amino group-CGA CGG ATC CCC GGG AAT TC (hereinafter referred to as "Pair 1-")
Amino group-AAC GGA ATC TAA TCA GGA GG (hereinafter referred to as "Pair 8+")
Amino group-CCT CCT GAT TAG ATT CCG TT (hereinafter referred to as "Pair 8-")
Amino group-CCG ACT ACA GAA GAG GAG AA (hereinafter referred to as "Pair 7+")

### EXAMPLE 2

### Preparation of Oligonucleotide-labeled IgG for Detection of Anti-HCV Antibody and TP

Pair 8- was introduced into a rabbit IgG. Specifically, 2 ml of 0.1 M sodium borate buffer containing 25 mg of the rabbit IgG was allowed to react with a 50- to 100-fold excess by mole of N-succinimidyl S-acetylthioacetate (SATA) (available from Pierce Chemical Company) with respect to IgG at 37 ° C for 1 hour. After the reaction, Tris-HCl buffer and hydroxylamine were added to the reaction mixture to a final concentration of 0.1 M, respectively, and the resulting mixture was allowed to react at 37° C for 1 hour. The mixture was then applied to a column filled with Sephadex G-25 (available from Amersham Pharmacia Biotech Company (Amersham Biosciences)) and thereby yielded an SH-introduced rabbit IgG. Separately, to 0.1 M 3-(N-morpholino)propanesulfonic acid (MOPS) buffer pH 7.0 containing 1000 nmol of Pair 8- dissolved therein and 5 mM EDTA, a 50-fold by mole excess of EMCS (available from Dojindo) with respect to the oligonucleotide was added and was allowed to react at 37 ° C for 1 hour. After the reaction, the resulting mixture was subjected to precipitation with ethanol and rinsing according to a conventional procedure and thereby yielded a maleimide-group-introduced oligonucleotide. The maleimide-group-introduced oligonucleotide was allowed to react with the SH-group-introduced IgG at 37 ° C for 1 hour, was then applied to a Ultrogel AcA 34 column(available from Biosepra) and thereby yielded a "Pair 8-"-labeled rabbit IgG.

### EXAMPLE 3

### Preparation of colloidal gold-labeled Streptavidin

A total of 5 ml of colloidal gold having particle diameter of 40 nm (absorbance at 530 nm) was added to and was allowed to react with 1 ml of 2 mM borate buffer pH 9.0 containing 150 µg of streptavidin (available from Vector Laboratories). The resulting mixture was blocked with bovine serum albumin with a final concentration of 1%, a colloidal gold-labeled streptavidin was then recovered as a precipitate by centrifugation, was subjected to centrifugal washing with 1% bovine serum albumin solution, the resulting precipitate was suspended in the solution and thereby yielded the target colloidal gold-labeled streptavidin.

### EXAMPLE 4

### Preparation of Oligonucleotide-labeled Streptavidin

To 0.1 ml of 0.2 M borate buffer pH 8.0 containing 2 mg of streptavidin dissolved therein, a 100-fold by mole excess of SATA with respect to the streptavidin was added and was allowed to react at 37° C for 1 hour. Tris buffer and hydroxylamine were then added to the resulting mixture to a final concentration of 0.1 M, respectively, and the resulting mixture was allowed to react at 37° C for 30 minutes. The reaction mixture was then applied to a Sephadex G-25 column and thereby yielded an SH-group-introduced streptavidin. Separately, to 0.1 M MOPS buffer pH 7.0 containing 216 nmol of Pair 8+ dissolved therein and 5 mM EDTA, a 100-fold by mole excess of EMCS with respect to the oligonucleotide was added and was allowed to react at 37° C for 1 hour. After the reaction, the resulting mixture was subjected to precipitation with ethanol and washing according to a conventional procedure and thereby yielded a maleimide-group-introduced oligonucleotide. The maleimide-group-introduced oligonucleotide was allowed to react with the SH-group-introduced streptavidin at 37 ° C for 1 hour, was then applied to a Ultrogel AcA 34 column and thereby yielded a "Pair 8+"-labeled streptavidin.

### EXAMPLE 5

### Preparation of HCV Core 3 Peptide-binding Colloidal gold-labeled Streptavidin

A total of 200 ml of the colloidal gold-labeled streptavidin prepared in Example 3 was added to 5 µl of a biotin-introduced hepatitis C virus (HCV) core 3 peptide (available from Innogenetics NV) and was allowed to react at 37 ° C for 1 hour. The resulting colloidal gold complex was recovered as a precipitate by centrifugation, was centrifugally washed with a 1% bovine serum albumin solution, the resulting precipitate was suspended in the solution, and thereby yielded HCV core 3 peptide-binding colloidal gold-labeled streptavidin.

### EXAMPLE 6

### Preparation of HCV Core 3 Peptide-binding Oligonucleotide-labeled Streptavidin

To 200 µg of the oligonucleotide-labeled streptavidin prepared in Example 4, 56 µg of the biotin-introduced HCV core 3 peptide was added and was allowed to react at 37 ° C for 1 hour, the resulting mixture was then subjected to ultrafiltration using YM30 (available from Millipore Corporation) and thereby yielded a HCV core 3 peptide-binding oligonucleotide-labeled streptavidin.

### EXAMPLE 7

### Preparation of "Pair 8-"-labeled IgG-binding Nitrocellulose Membrane

The "Pair 8-"-labeled rabbit IgG prepared in Example 2 was diluted to 1 mg/ml, was charged into XY3000 (the trade name of an XY dispensing system for the preparation of test strips, available from BioDot, Inc.) and was applied to a nitrocellulose membrane (available from Advanced Microdevices Pvt. Ltd. (mdi)) lined with poly ethylene terephthalate (PET). After drying, the resulting article was blocked with Blockace (the trade name of a blocking agent available from Snow Brand Milk Products Co., Ltd.) at room temperature for 3 hours, was washed with distilled water and was then dried. A cellulose filter paper (available from Whatman Inc. under the trade name of WF1.5 or from Advanced Microdevices Pvt. Ltd. (mdi)) was attached to one end of the dried article, and a conjugate release pad (a glass filter available from Advanced Microdevices Pvt. Ltd. (mdi)) to the other end. In this procedure, part of them were overlapped with each other. The resulting article was cut to 5 mm in width and was housed in a housing.

### EXAMPLE 8

### Reactivity in Anti-HCV Antibody Positive Serum

A total of 100 µl of an anti-HCV antibody positive serum was added to 11.5 µl of a solution containing 5 µl of the HCV core 3 peptide-binding colloidal gold-labeled streptavidin prepared in Example 5, 150 ng of the HCV core 3 peptide-binding oligonucleotide-labeled streptavidin prepared in Example 6, 4.4% Tween 80, and 44 mM EDTA, the resulting mixture was mixed and was immediately applied to a sample supply opening of the "Pair 8-"-labeled IgG-binding nitrocellulose membrane prepared in Example 7. A quarter of an hour later, a reddish violet line of the colloidal gold was observed in a portion where the "Pair 8-"-labeled IgG was applied. In contrast, no line was observed when a serum that turned out to contain no anti-HCV antibody was added as a control. These results revealed that the method of the present invention can determine the presence of a specific antibody to a ligand.

### EXAMPLE 9

### Preparation of colloidal gold-labeled TP Antigen

A total of 5 ml of colloidal gold having a particle diameter of 40 nm was mixed with and was allowed to react with 1 ml of 2 mM borate buffer pH 9.0 containing 100 µg of a genetically modified Treponema pallidum (TP) 17K antigen (available from Lee Laboratories). The resulting mixture was then blocked with bovine serum albumin with a final concentration of 1%, was subjected to centrifugation and thereby yielded a colloidal gold-labeled TP antigen as a precipitate. The recovered colloidal gold-labeled TP antigen was centrifugally washed with a 1% bovine serum albumin solution, the resulting precipitate was suspended in the solution and thereby yielded a colloidal gold-labeled TP antigen.

### EXAMPLE 10

### Preparation of Oligonucleotide-labeled Avidin

To 1 ml of 0.1 M phosphate buffer pH 7.0 containing 10 mg of avidin dissolved therein, a 100-fold by mole excess of EMCS with respect to the avidin was added and was allowed to react at 37° C for 1 hour. The resulting mixture was then applied to a Sephadex G-25 column and thereby yielded a maleimide-group-introduced avidin. Separately, to 0.1 M MOPS buffer pH 7.0 containing 375 nmol of Pair 8+ dissolved therein and 5 mM EDTA, a 100-fold by mole excess of SATA with respect to the oligonucleotide was added and was allowed to react at 37° C for 1 hour. After the reaction, Tris buffer and hydroxylamine were added to the resulting mixture to a final concentration of 0.1 M, respectively, and were allowed to react at 37° C for 30 minutes. The reaction mixture was then subjected to precipitation with ethanol and rinsing according to a conventional procedure and thereby yielded an SH-group-introduced oligonucleotide. The SH-group-introduced oligonucleotide was allowed to react with the maleimide-group-introduced avidin at 37 ° C for 1 hour, the resulting mixture was applied to Ultrogel AcA44 and thereby yielded "Pair 8+"-labeled avidin.

### EXAMPLE 11

### Preparation of Biotin-labeled TP 17K Antigen

To 0.5 ml of 0.1 M phosphate buffer pH 7.0 containing 500 µg of a TP 17K antigen (available from Innogenetics NV) and 5 mM EDTA, a 100-fold by mole excess of NHS-biotin (available from Pierce Chemical Company) with respect to the TP 17K antigen was added and was allowed to react at 37° C for 1 hour. After the reaction, the resulting mixture was applied to a Sephadex G-25 column and thereby yielded a biotin-labeled TP 17 antigen with a recovery of TP 17K antigen of 75%.

### EXAMPLE 12

### Preparation of Oligonucleotide-labeled TP 17K Antigen Through Reaction of Biotinylated TP 17K Antigen and Oligonucleotide-labeled Avidin

The oligonucleotide-labeled avidin prepared in Example 10 was mixed with the biotin-labeled TP 17K antigen prepared in Example 11 in a ratio of the former to the latter of 1:8, 1:4, 1:2, 1:1, and 1:0.5, respectively, and was allowed to react at 37° C for 30 minutes. The resulting mixtures were stored at 4 ° C.

In these mixtures, the recovery of the TP 17K antigen was 75% since they were prepared merely by mixing the oligonucleotide-labeled avidin prepared in Example 10 and the biotin-labeled TP 17K antigen prepared in Example 11.

### EXAMPLE 13

### Reactivity of Oligonucleotide-labeled TP 17K Antigen with Varying Different Mixing Ratios

The reactivity of each of the oligonucleotide-labeled TP 17K antigens with different mixing ratios prepared in Example 12 was evaluated. Specifically, 2 µl each of the oligonucleotide-labeled TP 17K antigens prepared in Example 12 was added to 5 µl of the colloidal gold-labeled TP antigen prepared in Example 9, 5 µl of 1 mg/ml biotin, and 90 µl of an anti-TP antibody negative serum, respectively. Two bottles of this reaction system were prepared, an anti-TP antibody positive serum was added only to one reaction system, and each of the two reaction systems was then rapidly mixed. The total amount of each of the resulting mixtures was applied to the sample supply opening of the housing including the "Pair 8-"-labeled IgG-binding nitrocellulose membrane prepared in Example 7.

Twenty minutes after the application, the positive specimen with a mixing ratio of 1:2 showed the darkest line, subsequently those with mixing ratios of 1:4 and 1:1 showed the darker lines to equal extent, followed by those with mixing ratios of 1:0.5 and 1:8 in this order. In contrast, specimens containing the anti-TP antibody negative serum with individual mixing ratios showed no line.

The result shows that the detection sensitivity can easily be changed by changing the mixing ratio of the oligonucleotide-labeled avidin to the biotin-labeled antigen.

### EXAMPLE 14

### Preparation of Oligonucleotide-labeled IgG for Detection of HBs Antigen

The oligonucleotide Pair 1- was introduced into a rabbit IgG in a similar manner to that in Example 2.

### EXAMPLE 15

### Preparation of Oligonucleotide-labeled Mouse Anti-HBs-Fab' for the Detection of HBs Antigen

To a 0.1 M citrate buffer pH 3.5 containing 10 mg of a monoclonal anti-HBs-IgG, pepsin (available from Boehringer Mannheim) was added to 5% of the IgG and was allowed to react at 37 ° C for a predetermined time. After the reaction, the resulting mixture was applied to a Ultrogel AcA44 column(trade name; available from Biosepra) equilibrated with 0.1 M phosphate buffer pH 6.0 to thereby collect an F(ab')₂ fraction.

A total of 5.2 mg of the above-obtained F(ab') ₂ was concentrated by ultrafiltration using YM30 (available from Millipore Corporation), the concentrate was reduced at 37° C for 90 minutes with mercaptoethylamine (available from Nakarai Tesque, Inc.) in a final concentration of 0.1 M. The resulting mixture was then applied to a Sephadex G-25 column (trade name, available from Amersham Pharmacia Biotech Company (Amersham Biosciences)) and thereby yielded 2.6 mg of a mouse anti-HBs-Fab'.

Separately, to 0.1 M MOPS buffer pH 7.0 containing 191 nmol of Pair 1+ dissolved therein and 5 mM EDTA, a 100-fold by mole excess of EMCS with respect to the oligonucleotide was added and was allowed to react at 37° C for 30 minutes. After the reaction, the resulting mixture was subjected to precipitation with ethanol and washing according to a conventional procedure and thereby yielded a maleimide-group-introduced oligonucleotide.

The maleimide-group-introduced oligonucleotide was allowed to react with the mouse anti-HBs-Fab' at 37° C for 1 hour, was then applied to a Ultrogel AcA44 column and thereby yielded " Pair 1+"-labeled mouse anti-HBs-Fab'.

### EXAMPLE 16

### Preparation of Nitrocellulose Membrane for the Simultaneous Detection of HBs Antigen and Anti-TP Antibody

The "Pair 8-"-labeled rabbit IgG prepared in Example 2 and the "Pair 1-"-labeled rabbit IgG prepared in Example 14 were diluted to a concentration of 1 mg/ml and were charged into XY3000 (trade name, available from BioDot, Inc.), respectively. The "Pair 8-"-labeled rabbit IgG and the "Pair 1-"-labeled rabbit IgG were applied to a nitrocellulose membrane lined with PET at positions of 35 mm and 30 mm from a starting point, respectively. After drying, the applied membrane was blocked with Blockace (trade name, available from Snow Brand Milk Products Co., Ltd.) at room temperature for 3 hours, was washed with distilled water and was then dried. A cellulose filter paper (available from Whatman Inc. under the trade name of WF1.5 or from Advanced Microdevices Pvt. Ltd. (mdi)) was attached to one end of the dried article, and a conjugate release pad (available from Advanced Microdevices Pvt. Ltd. (mdi)) to the other end. In this procedure, part of them were overlapped with each other. The resulting article was cut to 5 mm in width and was housed in a housing.

### EXAMPLE 17

### Analysis on Possibility of Simultaneous Detection of HBs Antigen and Anti-TP Antibody

The possibility of simultaneous detection of a HBs antigen and an anti-TP antibody was analyzed in the following manner. Specifically, the oligonucleotide-labeled TP 17K antigen with a mixing ratio of 1:4 prepared in Example 12 was diluted ten folds, and 1 µl of the diluted solution was added to 5 µl of the colloidal gold-labeled TP antigen prepared in Example 9, 5 µl of a biotin 1 mg/ml solution and 90 µl of an anti-TP antibody negative and HBs antigen negative serum. To the resulting mixture, 90 ng of the oligonucleotide-labeled mouse anti-HBs-Fab' prepared in Example 15 and 9 µl of a colloidal gold-labeled anti-HBs antibody (available from British Biocell International) were added. Four bottles of this reaction system were prepared. A total of 5 µl of an anti-TP antibody positive serum was added to one bottle, 5 µl of an anti-TP antibody negative and HBs antibody negative serum was added to another, and 5 µl of sera containing 50 ng/ml and 220 µg/ml of HBs antigen were added to the remained two bottles, respectively, and the resulting mixtures were immediately mixed, and the total amounts of the resulting mixtures were applied to the sample supply opening of the housing containing the "Pair 8-"-labeled rabbit IgG-binding and "Pair 1-"-labeled rabbit IgG-binding nitrocellulose membrane prepare in Example 16, respectively.

Twenty minutes after the application, the sample containing the anti-TP antibody negative and HBs antigen negative serum showed no line. The sample containing the anti-TP antibody positive serum showed a line only in a portion where the Pair 8- oligonucleotide was applied, and the samples each containing the HBs antigen showed a line only in a portion where the Pair 1- oligonucleotide was applied. Each of them was independently detected on the same nitrocellulose membrane.

### EXAMPLE 18

### Preparation of Biotin-labeled TP 17K Antigen

To 0.5 ml of 0.1 M phosphate buffer pH 7.0 containing 500 µg of TP 17K antigen (available from Lee Laboratories) and 5 mM EDTA, a 100-fold by mole excess of an NHS-biotin was added with respect to the TP 17K antigen and was allowed to react at 37° C for 1 hour. After the reaction, the resulting mixture was applied to a Sephadex G-25 column and thereby yielded a biotin-labeled TP 17K antigen with a recovery of the TP 17K antigen of 75%. The ultimate recovery of the biotin-labeled TP 17K antigen was 75%, since it was prepared merely by mixing the TP 17K antigen with the oligonucleotide-labeled avidin prepared in Example 10, as described in Example 12.

### COMPARATIVE EXAMPLE

### Preparation of Oligonucleotide-labeled TP 17K Antigen Through Direct Labeling of TP 17K Antigen

To 0.1 M phosphate buffer pH 7.4 containing 473 µg of a TP 17K antigen (available from Lee Laboratories) and 5 mM EDTA, a 20-fold by mole excess of SATA was added with respect to the TP 17K antigen and was allowed to react at 37 ° C for 90 minutes. Hydroxylamine and Tris buffer were added to and were allowed to react with the resulting mixture. The resulting reaction mixture was then applied to a Sephadex G-25 column (trade name, available from Amersham Pharmacia Biotech Company (Amersham Biosciences)) and thereby yielded 200 µg of an SH-group-introduced TP 17K antigen (recovery: 42.2%). Separately, to 0.1 M MOPS buffer pH 7.85 containing 133 nmol of Pair 7+ and 5 mM EDTA, a 200-fold by mole excess of EMCS with respect to the oligonucleotide Pair 7+ was added and was allowed to react at 37 ° C for 2 hours. The resulting oligonucleotide was subjected to precipitation with ethanol and rinsing according to a conventional procedure and thereby yielded 105 nmol of a maleimide-group-introduced oligonucleotide.

A total of 200 mg of the SH-group-introduced TP 17K antigen and 105 nmol of the maleimide-group-introduced oligonucleotide were mixed and were allowed to react with each other at 37° C for 2 hours. The resulting mixture was then applied to an Ultrogel AcA44 column and thereby yielded 150 µg of an oligonucleotide-labeled TP 17K antigen (recovery: 32%).

In comparison between Example 18 and Comparative Example, the product according to Comparative Example showed a recovery of 32%, and the product according to Example 18 showed a recovery of 75%, indicating that the recovery was significantly improved in Example 18.

### EXAMPLE 19

### Preparation of HCV Core 2, Core 3, Core 6 and Core 10-Binding colloidal gold-labeled Streptavidin

A total of 500 µl of a colloidal gold-labeled streptavidin (available from British Biocell International) prepared to show an absorbance at 530 nm of 10 was mixed with 2 µg of a biotinylated HCV core 2, 2 µg of a biotinylated HCV core 3, 2 µg of a biotinylated HCV core 6, and 2 µg of a biotinylated HCV core 10 (each available from Innogenetics NV) and was allowed to react at 37° C for 1 hour. After the reaction, a HCV core 2, core 3, core 6 and core 10-binding colloidal gold-labeled streptavidin was recovered as a precipitate by centrifugation. The precipitate was suspended in 20 mM sodium borate buffer pH 8.0 containing 2% bovine serum albumin and was stored at 4° C.

### EXAMPLE 20

### Oligonucleotide-labeled HCV Core 2, Core 3, Core 6, or Core 10-binding Streptavidin

To 100 µg of the "Pair 8+"-labeled streptavidin prepared in Example 4, 30 µg each of HCV core 2, core 3, core 6, or core 10 was added, respectively, was allowed to react at 37 ° C for 1 hour, the resulting mixtures were subjected to ultrafiltration and thereby yielded a "Pair 8+"-labeled HCV core 2-binding streptavidin, a "Pair 8+"-labeled HCV core 3-binding streptavidin, a "Pair 8+"-labeled HCV core 6-binding streptavidin, and a "Pair 8+"-labeled HCV core 10-binding streptavidin, respectively.

### EXAMPLE 21

### Preparation of Oligonucleotide-labeled HCV Core 2, Core 3, Core 6 and Core 10-binding Streptavidin

To 100 µg of the "Pair 8+"-labeled streptavidin prepared in Example 4, 30 µg each of HCV core 2, core 3, core 6 and core 10 were added concurrently and were allowed to react at 37 ° C for 1 hour. The resulting mixture was subjected to ultrafiltration and thereby yielded a "Pair 8+"-labeled HCV core 2, core 3, core 6 and core 10-binding streptavidin.

### EXAMPLE 22

### Reactivity with Anti-HCV antibody Positive Serum

The reactivity of the above-prepared articles was analyzed in the same manner as in Example 8. Specifically, a combination (Combination A) was prepared by mixing the HCV core 2, core 3, core 6 and core 10-binding colloidal gold-labeled streptavidin prepared in Example 19 with 37.5 ng of the Pair 8+"-labeled HCV core 2-binding streptavidin, 37.5 ng of the Pair 8+"-labeled HCV core 3-binding streptavidin, 37.5 ng of the Pair 8+"-labeled HCV core 6-binding streptavidin, and 37.5 ng of the Pair 8+"-labeled HCV core 10-binding streptavidin each prepared in Example 20. Another combination (Combination B) was prepared by mixing 5 µl of the HCV core 2, core 3, core 6 and core 10-binding colloidal gold-labeled streptavidin prepared in Example 19 with 150 ng of the "Pair 8+"-labeled HCV core 2, core 3, core 6 and core 10-binding streptavidin prepared in Example 21. The reaction with an anti-HCV antibody positive serum was observed 5 minutes and 7 minutes into the test in Combination A and in Combination B, respectively. In this connection, the reaction was observed 15 minutes into the test in Example 8. These results show that a reaction system including plural types of peptides bound to a labeled streptavidin exhibits higher reactivity than a reaction system including a single type of peptide bound to a labeled streptavidin.

### INDUSTRIAL APPLICABILITY

The kits and methods for detection or quantification of the present invention can control the sensitivity of reagents.

For example, the kits of the present invention can control the amount of the ligand L1 or the ligand L2 per molecule of the receptor I and receptor II. This configuration can control the sensitivity of measurement. Specifically, a substance having plural binding points to the substance B1 can be used as the substance R1, and thereby plural pieces of the ligand L1 can be bound to the substance R1 through the medium of the substance B1. Likewise, a substance having plural binding points to the substance B2 can be used as the substance R2, and thereby plural pieces of the ligand L2 or the ligand L3 can be bound to the substance R2 through the medium of the substance B2.

Specifically, when avidin or streptavidin is used as the substance R1 or R2, the avidin or streptavidin has four binding points per its molecule with respect to biotin as the substance B1, and the ratio of the introduced ligand L1 can be changed from one to four per molecule of avidin or streptavidin. The control of the inducing ratio can ensure the control of the reactivity and sensitivity. In addition, the sensitivity can be controlled by the compound of L-B1-R1-M and the compound of L-B1-R2-B3. ensuring the control of the sensitivity to further detail.

By selecting combinations of substances having different binding capabilities as the bond element B3 and the anti-bond element R3, the intensity of binding capability can be changed, and the sensitivity of reagents can be controlled.

When the bond element B3 and the anti-bond element R3 are oligonucleotides of RNA and/or DNA at least having partly complementary sequences and are bound to each other by action of their complementarity, the intensity of binding capability can be controlled, and thereby the sensitivity of the reagent can be controlled by changing the lengths and/or homology of the oligonucleotides.

In the kits of the present invention, the plural ligands to be bound to the substance R1 or R2 may have the same reactivity or may have plural types of reactivity. In the latter case, the resulting kit has higher reactivity to the analyte A than a kit including ligands having the same reactivity.

According to the kits and methods for detection or measurement of the present invention, when plural oligonucleotides of, for example, RNAs or DNAs are used as the bond element B3 and the anti-bond element R3 to be solid-phased, and the resulting complex is bound to the solid phase through at least partly complementary binding, oligonucleotides having different sequences can be used as the oligonucleotides. The invented kits and methods for detection or measurement can therefore simultaneously detect or quantify plural analytes corresponding to the plural types of the oligonucleotides.

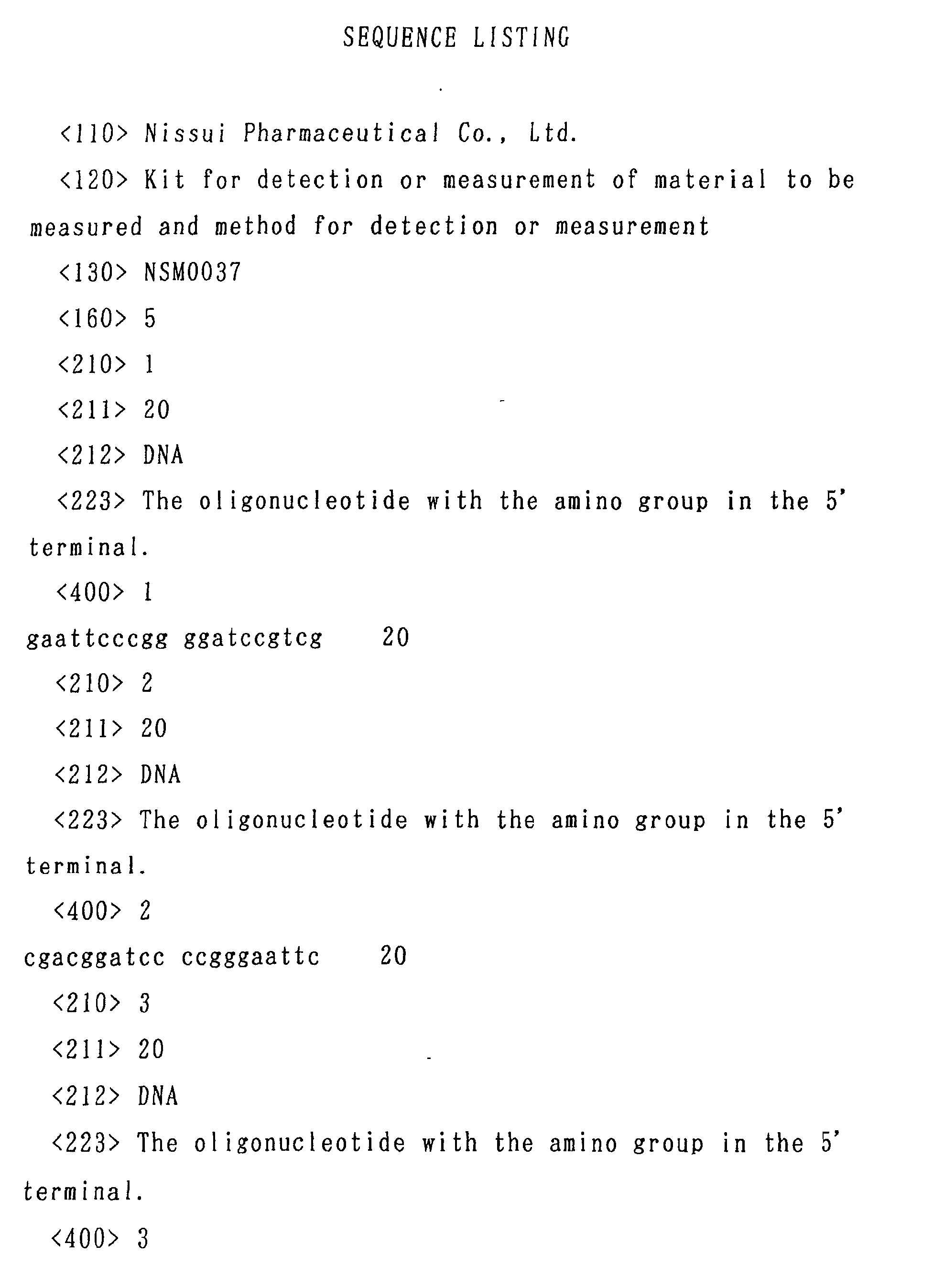

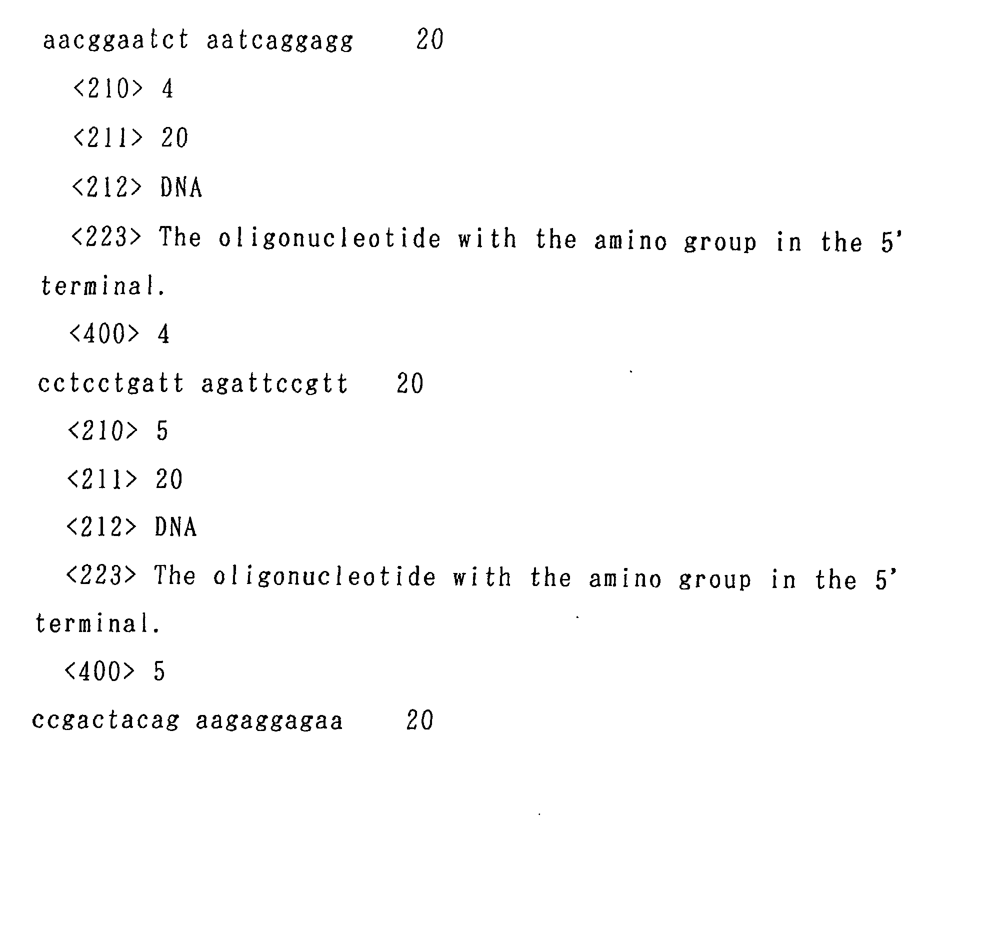

## Claims

1. A kit for detecting or measuring an analyte having bivalent or higher binding capability, the kit comprising the following receptor I, receptor II, and solid phase conjugate for capturing a complex comprising the receptor I, the receptor II and the analyte:
1) a receptor I represented by a compound L1-B1-R1-M comprising a compound R1-M bound to a compound L1-B1, the compound R1-M comprising a substance R1 bound to a marker M, the substance R1 being capable of binding to a substance B1, the compound L1-B1 comprising a ligand L1 bound to the substance B1, the ligand L1 being capable of binding to the analyte A;
2) a receptor II represented by a compound L2-B2-R2-B3 comprising a compound L2-B2 previously bound to a compound R2-B3, the compound L2-B2 comprising a ligand L2 and a substance B2 introduced into the ligand L2, the ligand L2 being capable of binding to the analyte A, the substance B2 having different binding capability from the analyte A, the compound R2-B3 comprising a substance R2 bound to a bond element B3, the substance R2 being capable of binding to the substance B2, the bond element B3 having binding capability different from the substance B2; and
3) solid phase conjugate represented by a compound R3-solid phase, the compound R3-solid phase comprising ananti-bond element R3 bound to a solid phase, the anti-bond element R3 being capable of binding to the bond element B3.

2. A kit for detecting or measuring an analyte A having bivalent or higher binding capability to a ligand L3 constituting part of a protein P, the kit comprising the following receptor I, receptor II, and solid phase conjugate for capturing a complex comprising the receptor I, the receptor II and the analyte:
1) a receptor I represented by a compound P-M comprising the protein P bound to a marker M;
2) a receptor II represented by a compound P-B2-R2-B3 or L3-B2-R2-B3 comprising a compound P-B2 or L3-B2 bound to a compound R2-B3, the compound P-B2 or L3-B2 comprising the protein P or the ligand L3 and a substance B2 introduced into the protein P or the ligand L3, the substance B2 having different binding capability from the analyte A, the compound R2-B3 comprising a substance R2 bound to a bond element B3, the substance R2 being capable of binding to the substance B2, the bond element B3 having binding capability different from the substance B2; and
3) solid phase conjugate represented by a compound R3-solid phase, the compound R3-solid phase comprising an anti-bond element R3 bound to a solid phase, the anti-bond element R3 being capable of binding to the bond element B3.

3. A kit for detecting or measuring an analyte A having bivalent or higher binding capability, the kit comprising the following receptor I, receptor II, and solid phase conjugate for capturing a complex comprising the receptor I, receptor II and analyte:
1) a receptor I represented by a compound L1-B1-R1-M comprising a compound R1-M bound to a compound L1-B1, the compound R1-M comprising a substance R1 bound to a marker M, the substance R1 being capable of binding to a substance B1, the compound L1-B1 comprising a ligand L1 bound to the substance B1, the ligand L1 being capable of binding to the analyte A;
2) a receptor II represented by a compound L2-B3, the compound L2-B3 comprising a ligand L2 previously bound to a bond element B3, the ligand L2 being capable of binding to the analyte A, the bond element B3 having different binding capability from analyte A; and
3) solid phase conjugate represented by a compound R3-solid phase, the compound R3-solid phase comprising an anti-bond element R3 bound to a solid phase, the anti-bond element R3 being capable of binding to the bond element B3.

4. The kit according to claim 1 or 3, wherein the substance R1 has a plurality of binding points with respect to the substance B1, and a plurality of the ligands L1 are bound to the substance R1 by the medium of the substance B1.

5. The kit according to claim 4, wherein each of the plurality of the ligands L1 has plural types of reactivity.

6. The kit according to claim 1 or 2, wherein the substance R2 has a plurality of binding points with respect to the substance B2, and a plurality of the ligands L2 or ligands L3 are bound to the substance R2 by the medium of the substance B2.

7. The kit according to claim 6, wherein each of the plurality of the ligands L2 or ligands L3 has plural types of reactivity.

8. The kit according to any one of claims 1 to 3, wherein the solid phase conjugate is at least independent of the receptor II.

9. The kit according to any one of claims 1 to 3, wherein the analyte A having bivalent or higher binding capability is a substance selected from the group consisting of DNAs, RNAs, antigens, and antibodies.

10. The kit according to any one of claims 1 to 3, wherein the ligand L1, ligand L2 or ligand L3 is a substance selected from the group consisting of DNA, RNA, antigen, antibody, lectin, glycoprotein, and sugar.

11. The kit according to claim 1 or 3, wherein the ligand L1 and the ligand L2 are the same substance.

12. The kit according to any one of claims 1 to 3, wherein the ligands L1 and L2 and the ligand L3 are substances having different sequences with one another.

13. The kit according to any one of claims 1 to 3, wherein the binding capability between the substance B1 and the substance R1 or between the substance B2 and the substance R2 is represented by a dissociation constant of from 10⁻⁸ to 10⁻¹⁶ (M).

14. The kit according to any one of claims 1 to 3, wherein the substance B1 and/or the substance B2 is biotin.

15. The kit according to any one of claims 1 to 3, wherein the substance B1 and/or the substance B2 is a substance selected from the group consisting of DNA, RNA, antigen, antibody, lectin, glycoprotein, and sugar.

16. The kit according to any one of claims 1 to 3, wherein the substance R1 and/or the substance R2 is a substance selected from the group consisting of streptavidin and avidin.

17. The kit according to any one of claims 1 to 3, wherein the substance R1 and/or the substance R2 is a substance selected from the group consisting of antigen, antibody, DNA, RNA, lectin, glycoprotein, and sugar.

18. The kit according to claim 1, wherein the substance R1 and the substance R2 are the same substance.

19. The kit according to claim 1, wherein the substance R1 and the substance R2 are different substances.

20. The kit according to any one of claims 1 to 3, wherein the bond element B3 is bondable to the anti-bond element R3 by complementary binding of at least part of DNAs or RNAs.

21. The kit according to any one of claims 1 to 3, wherein the marker M is a substance selected from the group consisting of coloring dye, fluorescent dye, luminescent substance, metal colloid, latex, liposome, radioactive isotope, enzyme, DNA, and RNA.

22. The kit according to any one of claims 1 to 3, wherein the solid phase is a substance selected from the group consisting of polystyrene, nitrocellulose, nylon, cellulose, and glass.

23. A method for detecting or measuring an analyte A having bivalent or higher binding capability, the method comprising the steps of:
bringing
1) the analyte A into contact with:
2) a receptor I represented by a compound L1-B1-R1-M comprising a compound R1-M bound to a compound L1-B1, the compound R1-M comprising a substance R1 bound to a marker M, the substance R1 being capable of binding to a substance B1, the compound L1-B1 comprising a ligand L1 bound to the substance B1, the ligand L1 being capable of binding to the analyte A; and
3) a receptor II represented by a compound L2-B2-R2-B3 comprising a compound L2-B2 previously bound to a compound R2-B3, the compound L2-B2 comprising a ligand L2 and a substance B2 introduced into the ligand L2, the ligand L2 being capable of binding to the analyte A, the substance B2 having different binding capability from the analyte A, the compound R2-B3 comprising a substance R2 bound to a bond element B3, the substance R2 being capable of binding to the substance B2, the bond element B3 having binding capability different from the substance B2, to react with one another to thereby form a complex;
4) allowing a solid phase conjugate to capture the formed complex, the solid phase conjugate represented by a compound R3-solid phase, the compound R3-solid phase comprising an anti-bond element R3 bound to a solid phase, the anti-bond element R3 being capable of binding to the bond element B3, and
5) detecting or measuring the marker M in the captured complex.

24. A method for detecting or measuring an analyte A having bivalent or higher binding capability to a ligand L3 constituting part of a protein P, the method comprising the steps of:
bringing
1) the analyte A into contact with:
2) a receptor I represented by a compound P-M comprising the protein P bound to a marker M; and
3) a receptor II represented by a compound P-B2-R2-B3 or L3-B2-R2-B3 comprising a compound P-B2 or L3-B2 bound to a compound R2-B3, the compound P-B2 or L3-B2 comprising the protein P or the ligand L3 and a substance B2 introduced into the protein P or the ligand L3, the substance B2 having different binding capability from the analyte A, the compound R2-B3 comprising a substance R2 bound to a bond element B3, the substance R2 being capable of binding to the substance B2, the bond element B3 having binding capability different from the substance B2, to react with one another to thereby form a complex,
4) allowing a solid phase conjugate to capture the formed complex, the solid phase conjugate represented by a compound R3-solid phase, the compound R3-solid phase comprising ananti-bond element R3 bound to a solid phase, the anti-bond element R3 being capable of binding to the bond element B3, and
5) detecting or measuring the marker M in the captured complex.

25. A method for detecting or measuring an analyte A having bivalent or higher binding capability, the method comprising the steps of:
bringing
1) the analyte A into contact with:
2) a receptor I represented by a compound L1-B1-R1-M comprising a compound R1-M bound to a compound L1-B1, the compound R1-M comprising a substance R1 bound to a marker M, the substance R1 being capable of binding to a substance B1, the compound L1-B1 comprising a ligand L1 bound to the substance B1, the ligand L1 being capable of binding to the analyte A; and
3) a receptor II represented by a compound L2-B3, the compound L2-B3 comprising a ligand L2 previously bound to a bond element B3, the ligand L2 being capable of binding to the analyte A, the bond element B3 having different binding capability from analyte A, to react with one another to thereby form a complex,
4) allowing a solid phase conjugate to capture the formed complex, the solid phase conjugate represented by a compound R3-solid phase, the compound R3-solid phase comprising ananti-bond element R3 bound to a solid phase, the anti-bond element R3 being capable of binding to the bond element B3, and
5) detecting or measuring the marker M in the captured complex.
